Europäisches Patentamt

European Patent Office

Office européen des brevets

(11) Publication number: **0 309 092 B1**

# EUROPEAN PATENT SPECIFICATION

(45) Date of publication of patent specification: **09.12.92**   (51) Int. Cl.5: **A61M 5/00**, A61M 25/00

(21) Application number: **88307685.3**

(22) Date of filing: **19.08.88**

(54) **Implantable device.**

(30) Priority: **25.08.87 US 89113**

(43) Date of publication of application:
**29.03.89 Bulletin 89/13**

(45) Publication of the grant of the patent:
**09.12.92 Bulletin 92/50**

(84) Designated Contracting States:
**AT BE CH DE ES FR GB GR IT LI LU NL SE**

(56) References cited:
**EP-A- 0 230 747**
**EP-A- 0 233 986**
**WO-A-87/01041**
**US-A- 3 310 051**

(73) Proprietor: **INFUSAID, INC.**
**1400 Providence Highway**
**Norwood Massachusetts 02062(US)**

(72) Inventor: **Johnston, Jimmie Tom**
**15 Victoria Circle**
**Norwood, Massachusetts(US)**
Inventor: **Sampson, Edward Jennings**
**315 Fiske Street**
**Carlisle, Massachusetts(US)**

(74) Representative: **Bradbrook, Geoffrey William**
**et al**
**PFIZER LIMITED Ramsgate Road**
**Sandwich Kent, CT13 9NJ(GB)**

## Description

This invention relates to an implantable device and in particular, to a vascular access device that is implanted subcutaneously.

Within the prior art, a variety of implantable access devices are known. Typical is the commercially available INFUSAID Infuse-A-Port™. These techniques of providing access via a various implantable device include percutaneous catheters, implantable ports having access to a port at a perpendicular angle to the skin and direct access with a needle. Thus, in the case of the commercially available Infuse-A-Port™, a base having an inlet located under the skin having an access outlet perpendicular to the skin line. The catheter thus extends at a right angle to the direction of needle access to the port's inlet.

A hallmark characteristic of many previous techniques of access utilizing implantable ports is a requirement that a needle be placed into the port septum at a 90° angle to the outlet catheter. This is acceptable for bolus injections or infusions over brief periods of time. However, for longer infusions or for continuous infusions with these ports, a right angle needle is required to allow for the hub of the needle to be parallel with the skin. This is required to permit anchoring of the needle to the body during infusions.

Another disadvantage with such prior art devices is that they require minor but, a distinct surgical procedure for implantation. That is, the size of the base is such that a significant incision is required for implantation. Moreover, given the size of the base, implantation is required in specific portions of the body, for example, the chest and stomach area that can physically support and house the port without protuberances or discomfort to the patient.

EP-A1-0 233 986 discloses an implantable access device with the axis of the front extending from the inlet to the outlet substantially parallel to the skin line. The allignment at the middle has to be precise.

Given these deficiencies of prior art devices, it is an object of this invention to define an implantable access device for humans having a low profile capable of implantation in a variety of bodily locations, which provides access to multiple body sites.

Yet another object of this invention is to define a low, acute angle implantable port which provides access to multiple body sites for research purposes in animals.

Yet another object of this invention is to provide an acute angle implantable device providing access to multiple body sites in small patients, such as infants, neonates and children.

A further object of this device is to provide for an implantable port which has a reduced size such that implantation can be carried out minimizing both the surgical procedure time and size of the incision.

These and other objects of this invention are achieved by means of an implantable port having a hollow port body, an inlet holding a self-sealing septum, and an outlet connected to a catheter. The port body has grooves, wings, or flaps which allow for suturing the port to subcutaneous tissue. The self-sealing septum in accordance with this invention is positioned at substantially a right angle to the outlet such that a needle puncturing the septum will be substantially in line with the outlet. This provides direct access to the catheter, allows for catheter tip placement or for the management of blockages in the catheter. Such is extremely difficult in the context of ports which have inlets disposed at right angles to the catheter outlet.

These and other objects of this invention are set forth herein by reference to the attached drawings and the descriptions of the preferred embodiments which follows.

Figure 1 is a schematic perspective view of a first embodiment of an implantable port in accordance with this invention;

Figure 2 is a schematic illustration showing the placement of the implantable port of this invention subcutaneously with the catheter extended into a blood vessel;

Figure 3 is a schematic cut-away view of a second embodiment of this invention;

Figure 4 is a perspective view of the embodiment of Figure 3 utilizing a separately molded tie-down element;

Figure 5 is an end view of the tie-down element of Figure 4;

Figure 6 is a schematic cut-away view of a third embodiment of this invention;

Figure 7 is a schematic cut-away side view of a fourth embodiment of this invention;

Figure 8 is a schematic cut-away side view of a fifth embodiment of this invention; and

Figure 9 illustrates a modified septum.

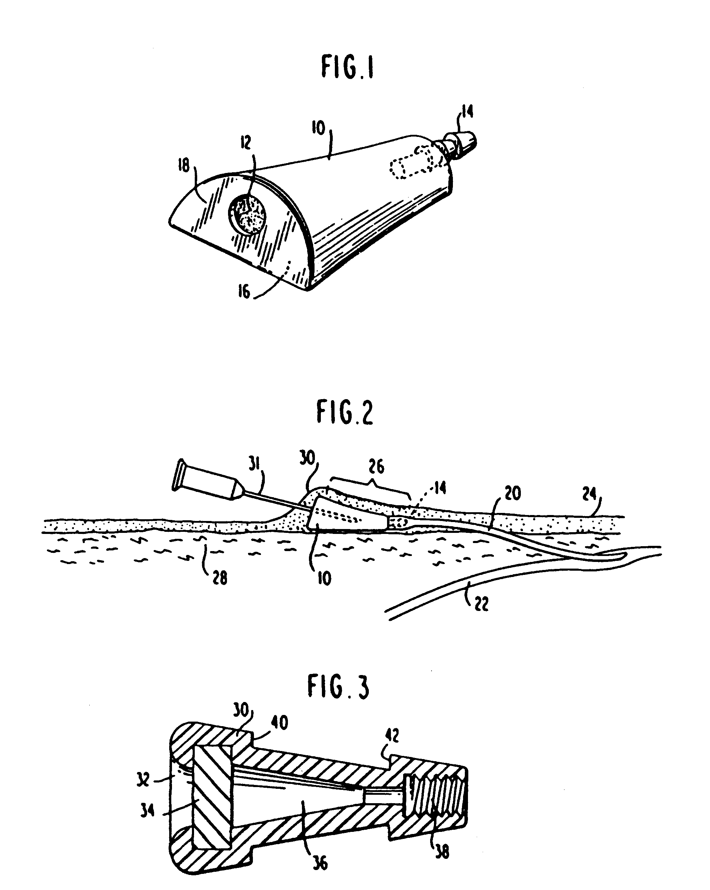

Referring now to Figure 1, a first embodiment of the implantable port of this invention is illustrated. The port comprises a body member 10 having a self-sealing septum 12 and an outlet connecter 14 to which a suitable catheter is mounted. As illustrated in Figure 1, the port has a generally flat base area 16 with an inclined face 18 into which the septum is placed. The septum provides a small diameter target, typically 0.15-0.20 inches. The overall length of the device from the front face to the tip of the connecter is in the range of one inch. The overall height of the device from the flat base 16 to the tapered top portion is at a maximum approximately 0.38 inches. The port and the connecter are manufactured from a biocompatible material. Materials of choice for the port are silicone rubber and various plastics such as PVC, teflon, polyethylene, polypropylene, polyurethane, polycarbonate, polyethersulfone, polysulfone, polyolefin, nylon, and the like. The connecter is preferably a metallic member made of stainless steel or titanium. The entire device can be made of one piece, for example a suitable metal.

As illustrated in Figure 1, the front face 18 is inclined so that access to the self-sealing septum 12 is axially aligned with the connecter 14. This is in contrast to prior art systems wherein normal access to the septum would be disposed at a right angle, that is perpendicular to the outlet connecter to the catheter.

Given the low profile of the device, implantation in areas with limited subcutaneous tissue such as forearms, scalp and neck area, infants and children and their appendages is possible.

Referring now to Figure 2, the device of Figure 1, including the catheter, is shown implanted. Specifically, the port 10 has coupled to it a catheter 20 of suitable length. The catheter is force-fitted onto the connecter 14 having its free end lanced into a suitable blood vessel 22. The catheter made from a biocompatible material such as silicone rubber or polyurethane and depending on the application may have a radiopaque material added. The device is implanted under the skin 24 by making a small localized incision 26.

The incision is shallow and does not involve incursion into underlying muscle tissue 28. Given the low profile of the device, a small protrusion 30 in the skin is present but such is not obtrusive or acts in any way as an impediment to normal functioning at the implantation site.

Access to the device 10 is by means of a needle 31. As can be seen from Figure 2. The needle penetrates the skin at the protrusion 30 directly into the target or port zone 12 and is in line with the outlet 14. A major advantage of this system is that given the in-line nature of the septum, outlet and catheter access to the catheter tip for management of blockages is possible. This also allows the use of straight needles as illustrated in Figure 2 for access to the port since entry is generally parallel to the skin line. Moreover, if it is necessary to pass a guide wire through the port and into the catheter, such can be done without making any significant bends. The ability to pass a guide wire or other appropriate device into the catheter after implantation provides a significant advantage in terms of clearing the catheter or importantly, initially placing the catheter tip at an appropriate location within the body. Such is extremely difficult in prior art right angle systems.

As can be appreciated from Figure 2, the use of a straight needle provides a material advantage over prior techniques. Additionally, if the needle is required to be taped down then, it is a simple matter of affixing the needle when in contact with the septum to the skin yet not substantially immobilize the patient. Thus, for example, if the port 10 is implanted in an arm the needle can simply be taped or strapped in place thereby anchoring the needle to the body during infusion or injections requiring a period of time.

Figure 3 illustrates a second embodiment of an in-line port in accordance with this invention. As illustrated in Figure 3, the port is generally conical and comprises the body member 30 having an opening 32. A self-sealing septum 34 is placed into the body 30 to close off the opening 32 and provide a suitable target. In this embodiment, the septum is a circular disk having a flat target face for the needle. The port has a hollow portion 36 generally axially in line with the self-sealing septum 34. The hollow portion serving as a reservoir terminates into a zone which is tapped, that is, area 38 to allow for the catheter connection. The overall length of the device is in the range of 0.75-1.0 inches. The maximum diameter is in the range of 0.4 inches. The exposed area, that is, opening 32 is in the range of 0.25 inches. As in the case of the first embodiment a variety of materials may be used, such as polysulfone.

The device of Figure 3 has two annular shoulders 40 and 42. These shoulders provide zones for holding a tie-down element (not illustrated) around the device.

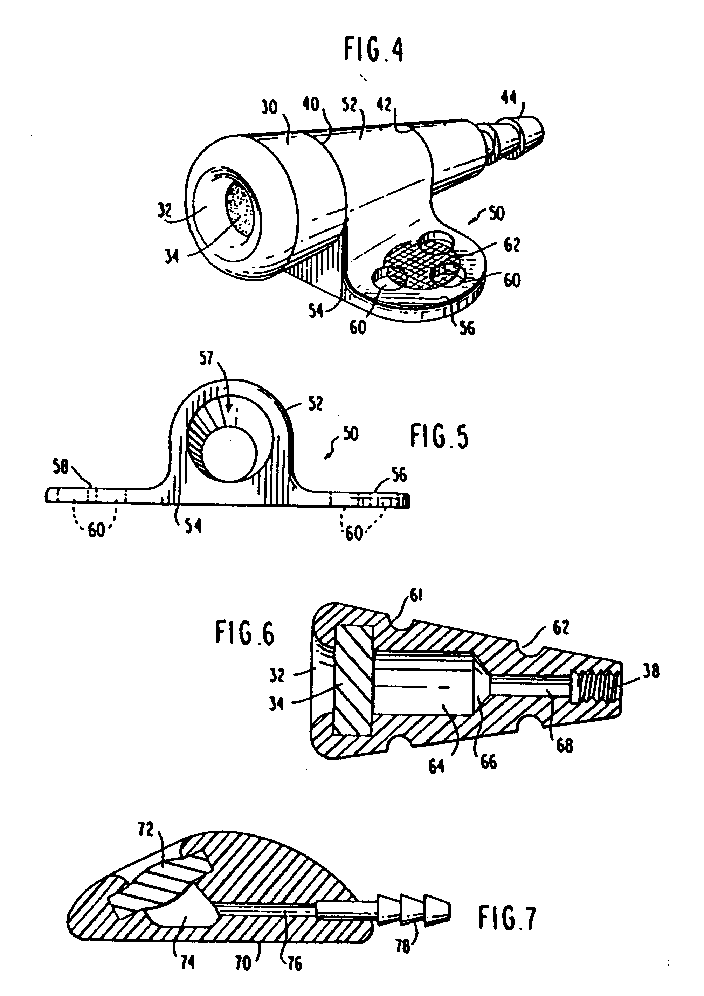

Referring now to Figures 4 and 5, a perspective view of the port illustrated in Figure 3 is depicted. The numeral is used to identify the same aspects of the embodiment of Figure 3 are used in Figures 4 and 5. Additionally, Figure 4 illustrates the connecter 44 for the catheter coupled to the outlet portion of the port, that is, screwed into the zone 38 and having a series of annular barbs or serrations upon which the catheter is fixed.

As illustrated in Figure 5 the tie-down comprises a separate element which is a separately molded material. Specifically, the tie-down 50 comprises a body portion 52 inclined to the horizontal relative to a base portion 54. Through an opening 57, the device 30 is inserted such that as illustrated in Figure 4 the rear portion of the device at shoulder 30 butts against the rear portion of the tie-down while the front wall. Consequently, when snapped into position the tie-down inclines the port and provides a pair of extending "wings" for purposes of suturing the device into place. Various techniques of suturing the wings 56, 58 may be used. As illustrated in Figure 4, a series of holes 60 can allow for sutures to be stitched through and around each of the wings. Alternatively, a zone of exposed dacron fabric 62 may be used to provide a confined anchoring area of each of the wing surfaces.

Referring now to Figure 6, a third preferred embodiment of this invention is illustrated. The embodiment of Figure 6 departs from that illustrated in Figure 3 in that the device while retaining its generally truncated conical form eliminates he shoulder zones 40 and 42. In its place, two annular rings 60 and 62 are employed. These provide two suture hold down locations. Another variation is that the hollow area 64 in the embodiment of Figure 6 is generally rectangular and has a truncated zone 66 coupling a straight in-line portion 68 to the hollow area 64. This serves as a guide for the needle, it being understood that the needle would generally terminate in the zone 64 providing direct fluid access to the catheter. The barbed connector which would be screwed into the device and the threaded portion 38.

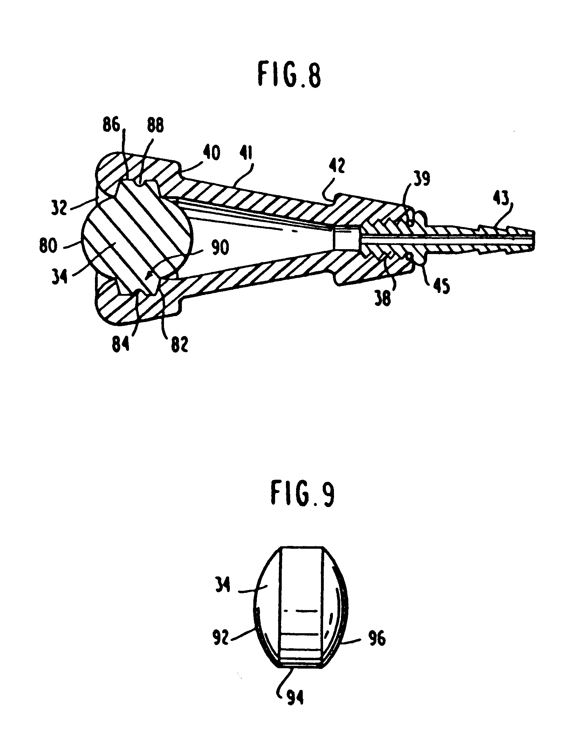

Referring now to Figure 7, a fourth embodiment of this invention is depicted. In Figure 7, the device comprises a generally squat body portion 70 having a self-sealing septum 72 embedded therein. The septum 72 is inclined relative to the horizontal flat bottom portion of the device and has a cavity portion 74 defined within the body 70. An outlet hollow port 76 is in fluid communication with an external connecter piece 78. The connecter piece is force-fitted or the like into the body portion 70 and serves as the connector between the port and the catheter. The body portion may be made of polysulfone or the like and, as illustrated in Figure 7 has a very low profile. Needle access is provided at a shallow, acute angle to the self-sealing septum 72. The device is anchored in place by through-holes, wings or the like which are not illustrated.

Referring to Figures 8 and 9, a fifth embodiment of this invention is depicted. This embodiment is generally similar to that of Figure 3 with the exception that the self-sealing septum has been modified. To the extent that common numerals are used in describing the port body as in Figure 3, such have been used in Figure 8. Thus, the port is generally conical and comprises a body having an opening 32. A pair of annular shoulders 40 and 42 provides zones for holding a tie-down element around the device. Alternatively, the recess 41 allows for the use of the wing tie-down as illustrated in Figures 4 and 5. The outlet 38 is tapped into the body and has, if necessary, an O-ring seal 39. The catheter connection 43 extends beyond the body. That catheter connection may, also have a shoulder 45 which defines a positive stop limiting insertion of the catheter connection into the port body.

In accordance with this embodiment of the invention, the self-sealing septum 34 comprises a generally spherical body having a dome-shaped striking face 80. The septum 34 also has an annular ring 82. The ring 82 further comprises an annular recess 84. The opening 32 of the port comprises compatible structure to lock the septum into place. Thus, as illustrated in Figure 3, the port comprises an annular recess 86 which engages the annular ring 82 on the septum. The recess 86 has an annular protrusion 88 which engages the annular recess 84 on the septum. This defines a positive lock zone 90.

This embodiment offers improvements over the septum of Figures 3 or 6 in that the increased depth of the septum, given its spherical shape, provides increased needle holding force to resist both pull out and provide necessary lateral rigidity. Additionally, the septum is easier to install due to its own rounded shape and because of the groove in the port body. The septum is inserted until the protrusion 88 engages the recess 84, thereby defining a positive locking in the recess 86.

Figure 9 illustrates a modified ball-shaped septum of Figure 8. This septum has the same outside diameter as the device of Figure 8, however, the sphere is truncated to reduce overall thickness. Consequently, as illustrated in Figure 9, the septum 34 has a convex striking face 92 and a generally flat circular section 94. That flat circular section would engage the groove of the port body as illustrated in Figure 3. The septum also has a convex-shaped rear face 96. This device offers the same improvements in terms of increased needle holding force for both pull out and lateral rigidity, but requires less force to insert into the port body. That is, the circular section defines a flat high pressure ridge around the septum to provide a flange effect of dome-shape with respect to the front and rear faces 92 and 94. While not illustrated, if necessary, the compatible protrusions and grooves as illustrated in Figure 8 may be incorporated into this modification.

It is apparent that while multiple embodiments of this invention have been illustrated, various other modifications can be made without departing from the essential scope of the invention. A key aspect is the geometry of the device so that straight needles can be used with the device implanted at a very shallow implantation site within an arm, the neck area or the like. Such is considered a material advantage over prior art systems which must be implanted in the torso given their overall size and geometry.

## Claims

1. An implantable access device comprising a hollow port (10) having an inlet (18) and an outlet (14), and a self-sealing septum (12) positioned on said port transversely to said outlet, the outlet having means for mounting a catheter, the axis of the port extending from the inlet to the outlet substantially parallel to the skin line in use **characterised** in that it comprises an infusions chamber between the inlet and the outlet, the walls of the infusion chamber converging from the inlet to the outlet, such that a straight needle puncturing said septum may be substantially in line with said outlet and guided towards the outlet by said converging walls, the port having anchoring means extending along the port between the inlet and outlet to facilitate anchoring of the device immediately below the skin.

2. The device of Claim 1 having a flexible catheter (20) mounted on said mounting means to provide fluid access to a portion of the body in which said device is implanted.

3. The device of Claim 2 characterized in that said means to mount a catheter comprises a hollow connector one end of which is inserted into said outlet of said port and the other end having annular barbs engaging said catheter.

4. The implantable device of Claim 1, 2 or 3 characterized in that said anchoring means comprises first and second shoulder portions (40, 42) provided on said port, said shoulder portions extending circumferentially around said port.

5. The implantable device of Claim 1, 2 or 3 characterized in that said anchoring means comprises first and second circumferential recesses (61, 62) provided on said port.

6. The implantable device of Claim 1, 2 or 3 characterized in that said anchoring means comprises a tie-down member (50) having means to mount said body, said tie-down member further including a flat portion (56, 58) defining a sutures area.

7. The implantable device of any preceding Claim, characterized in that said self-sealing septum (72) is angled at a shallow acute angle with respect to said outlet.

8. The device of any preceding Claim, further characterized in that said self-sealing septum comprises a generally spherically shaped body (80) having an annular ring (82), said annular ring engaging said hollow port.

9. The device of Claim 8 further characterized in that said port further comprises an annular recess (86) having an annular protrusion (88) and defining said inlet, said septum having an annular groove in said annular ring engaging said annular protrusion to lock said septum in place in said inlet.

10. The device of any one of Claims 1 to 7, further characterized in that self-sealing septum comprises a generally cylindrical body (94) having a convex front face (92).

**Patentansprüche**

1. Implantierbare Zugangseinrichtung, umfassend eine hohle Porteinrichtung (10) mit einem Eintritt (18) und einem Austritt (14) und ein selbstverschließendes Septum (12), das an der Porteinrichtung gegenüber dem Austritt angeordet ist, wobei der Austritt Mittel zum Montieren eines Katheters aufweist, die Achse der Porteinrichtung vom Eintritt zum Austritt im wesentlichen paralell zur Hautlinie verläuft, bei Gebrauch dadurch gekennzeichnet, daß sie eine Infusionskammer zwischen dem Eintritt und dem Austritt umfaßt, wobei die Wände der Infusionskammer vom Eintritt zum Austritt konvergieren, so daß eine gerade, das Septum durchstechende Nadel im wesentlichen mit dem Austritt übereinstimmt und durch die konvergierenden Wände in Richtung des Austritts geführt wird, wobei die Porteinrichtung Mittel zum Befestigen aufweist, die entlang der Porteinrichtung zwischen dem Eintritt und Austritt verlaufen, um eine Befestigung der Einrichtung unmittelbar unter der Haut zu erleichtern.

2. Einrichtung nach Anspruch 1 mit einem auf dem Montageelement montierten flexiblen Katheter (20), um einer Flüssigkeit Zugang zu einem Abschnitt des Körpers, in den die Einrichtung implantiert wurde, zu gewähren.

3. Einrichtung nach Anspruch 2, dadurch gekennzeichnet, daß das Element zum Montieren des Katheters eine hohles Verbindungselement umfaßt, dessen eines Ende in den Austritt der Porteinrichtung eingesetzt ist und dessen anderes Ende ringförmige Widerhaken aufweist, die den Katheter festhalten.

4. Implantierbare Einrichtung nach Anspruch 1, 2 oder 3, dadurch gekennzeichnet, daß das Befestigungsmittel erste und zweite Vorsprungsabschnitte (40, 42) umfaßt, die auf der Porteinrichtung vorgesehen sind und um den Umfang der Porteinrichtung herumverlaufen.

**5.** Implantierbare Einrichtung nach Anspruch 1, 2 oder 3, dadurch gekennzeichnet, daß das Befestigungsmittel erste und zweite, auf der Porteinrichtung vorgesehene Kreisumfangsaussparungen (61, 62) umfaßt.

**6.** Implantierbare Einrichtung nach Anspruch 1, 2 oder 3, dadurch gekennzeichnet, daß das Befestigungsmittel ein Befestigungselement (50) umfaßt, das Mittel zum Montieren des Gehäuses aufweist, wobei das Befestigungselement ferner einen flachen, ein Nahtgebiet definierenden Abschnitt (56, 58) einschließt.

**7.** Implantierbare Einrichtung nach irgendeinem vorhergehenden Anspruch, dadurch gekennzeichnet, daß das selbstverschließende Septum (72) in einem flachen spitzen Winkel zum Austritt gewinkelt ist.

**8.** Einrichtung nach irgendeinem vorhergehenden Anspruch, die ferner dadurch gekennzeichnet ist, daß das selbstverschließende Septum einen im allgemeinen kugelig geformten Körper (80) mit einem kreisförmigen Ring (82), der in die hohle Porteinrichtung eingreift, umfaßt.

**9.** Einrichtung nach Anspruch 8, die ferner dadurch gekennzeichnet ist, daß die Porteinrichtung auch eine ringförmige Aussparung (86) mit einem ringförmigen, den Eintritt definierenden Vorsprung (88) umfaßt, wobei das Septum eine ringförmige Furche im kreisförmigen, in den ringförmigen Vorsprung eingreifenden Ring aufweist, um das Septum am Eintritt an seiner Stelle zu arretieren.

**10.** Einrichtung nach irgendeinem der Ansprüche 1 bis 7, die ferner dadurch gekennzeichnet ist, daß das selbstverschließende Septum einen im allgemeinen zylindrischen Körper (94) mit einer konvexen Vorderseite (92) umfaßt.

**Revendications**

**1.** Dispositif d'accès implantable comportant un embout creux (10) ayant une entrée (18) et une sortie (14), et une cloison auto-obturatrice (12) placée sur ledit embout transversalement à ladite sortie, la sortie ayant des moyens pour le montage d'un cathéter, l'axe de l'embout s'étendant de l'entrée à la sortie sensiblement parallèlement à la ligne de peau pendant l'utilisation, caractérisé en ce qu'il comporte une chambre à perfusion entre l'entrée et la sortie, les parois de la chambre à perfusion convergeant de l'entrée vers la sortie de manière qu'une aiguille droite perforant ladite cloison puisse être sensiblement alignée avec ladite sortie et guidée vers la sortie par lesdites parois convergentes, l'embout ayant des moyens d'ancrage s'étendant le long de l'embout entre l'entrée et la sortie pour faciliter l'ancrage du dispositif immédiatement sous la peau.

**2.** Dispositif selon la revendication 1 ayant un cathéter flexible (20) monté sur lesdits moyens de montage pour établir un accès de fluide à une partie du corps dans laquelle ledit dispositif est implanté.

**3.** Dispositif selon la revendication 2, caractérisé en ce que lesdits moyens pour le montage d'un cathéter comprennent un raccord creux dont une extrémité est insérée dans ladite sortie dudit embout et dont l'autre extrémité comporte des arêtes annulaires engageant ledit cathéter.

**4.** Dispositif implantable selon la revendication 1, 2 ou 3, caractérisé en ce que lesdits moyens d'ancrage comprennent des première et seconde parties épaulées (40, 42) prévues sur ledit embout, lesdites parties épaulées entourant circonférentiellement ledit embout.

**5.** Dispositif implantable selon la revendication 1, 2 ou 3, caractérisé en ce que lesdits moyens d'ancrage comprennent des premier et second évidements circonférentiels (61, 62) prévus sur ledit embout.

**6.** Dispositif implantable selon la revendication 1, 2 ou 3, caractérisé en ce que lesdits moyens d'ancrage comprennent un élément de bridage (50) ayant des moyens pour un montage sur ledit corps, ledit élément de bridage comprenant en outre une partie plate (56, 58) définissant une zone de sutures.

**7.** Dispositif implantable selon l'une quelconque des revendications précédentes, caractérisé en ce que ladite cloison auto-obturatrice (72) est inclinée de façon à former un petit angle aigu avec ladite sortie.

**8.** Dispositif selon l'une quelconque des revendications précédentes, caractérisé en outre en ce que ladite cloison auto-obturatrice comprend un corps (80) de forme globalement sphérique ayant une bague annulaire (82), ladite bague annulaire engageant ledit embout creux.

**9.** Dispositif selon la revendication 8, caractérisé en outre en ce que ledit embout présente en outre un évidement annulaire (86) ayant une saillie annulaire (88) et définissant ladite entrée, ladite cloison ayant une gorge annulaire dans ladite bague annulaire engageant ladite saillie annulaire pour bloquer ladite cloison en place dans ladite entrée.

10. Dispositif selon l'une quelconque des revendications 1 à 7, caractérisé en outre en ce que la cloison auto-obturatrice comprend un corps globalement cylindrique (94) ayant une face avant convexe (92).

## FIG.1

## FIG.2

## FIG.3

FIG.4

FIG.5

FIG.6

FIG.7

## FIG.8

## FIG.9